# EUROPÄISCHE PATENTSCHRIFT

(11) **EP 2 683 318 B1**
(45) Veröffentlichungstag und Bekanntmachung des Hinweises auf die Patenterteilung: **13.04.2016**
(21) Anmeldenummer: 12703070.8
(22) Anmeldetag: 02.02.2012
(51) Int. Cl.: A61C 3/03, A61C 1/07, B06B 3/00

(54) **SONOTRODE FÜR DIE EINBRINGUNG VON ULTRASCHALL-ENERGIE**
SONOTRODE FOR THE INTRODUCTION OF ULTRASONIC ENERGY
SONOTRODE POUR LA TRANSMISSION D'ÉNERGIE ULTRASONORE

(30) Priorität: 11.03.2011 CH 419112011
(43) Veröffentlichungstag der Anmeldung: 15.01.2014
(73) Patentinhaber: Nexilis AG bei BDO AG, 2540 Grenchen (CH)
(72) Erfinder: STOFFEL, Marco, 8143 Stallikon (CH); WERNER, Uwe, 8825 Hütten (CH)
(74) Vertreter: Bremi, Tobias Hans
(86) Internationale Anmeldenummer: PCT/EP2012/051768
(87) Internationale Veröffentlichungsnummer: WO 2012/123182

(56) Entgegenhaltungen:
- JP-A- 63 037 923
- US-A1- 2004 112 547
- US-A1- 2006 253 050
- US-A1- 2008 132 927
- US-A1- 2009 047 623
- US-A1- 2010 130 867
- US-A1- 2010 179 654

## Beschreibung

### TECHNISCHES GEBIET

Die vorliegende Erfindung betrifft Sonotroden für die Einbringung von Ultraschall-Energie insbesondere in poröse Ausnehmungen, Sonotrodenwerkzeuge mit derartigen Sonotroden sowie Verwendungen von solchen Vorrichtungen.

### STAND DER TECHNIK

Ultraschall wird in zunehmendem Masse dazu eingesetzt, Verbindungen zwischen Werkstücken herzustellen, indem sich unter Ultraschalleinwirkung verflüssigendes Material in eine Verbindungsstelle eingetragen wird, diese unter Ultraschall verflüssigt wird, und anschliessend eine formschlüssige und/oder stoffschlüssige Verbindung zwischen zwei Werkstücken etabliert.

Damit die Verflüssigung möglichst effizient, d.h. unter Verwendung von möglichst wenig Schwingungsenergie, und möglichst lokalisiert stattfindet, ist es nötig, Vorrichtungen bereit zu stellen, die es ermöglichen, die Ultraschallschwingungen mit einer für den entsprechenden Anwendungsfall optimalen Orientierung der Ultraschallschwingung einzubringen. Zudem ist es wichtig, auch den häufig schwierigen Platzbedingungen bei der Einbringungsstelle gerecht zu werden, indem eine Umlenkung der Schwingungen realisiert wird.

Entsprechend gibt es, aus dem medizinischen wie auch aus dem nicht-medizinischen Bereich, eine Vielzahl von speziellen Vorrichtungen, so genannten Sonotrodenwerkzeugen, welche die Einbringung von Ultraschalls ermöglichen.

Dabei wird, wie beispielsweise aus der US 5,100,321, der US 2003/0157458, der EP 0 535 542 oder der US 5,899,693 resp. der EP 1 530 953 eine Umlenkung des Ultraschalls in den Anwendungsbereich, soweit in diesen Fällen überhaupt von einer tatsächlichen gezielten Umlenkung des Ultraschalls gesprochen werden kann, gewährleistet, indem auf ein beim Ultraschall erzeugendes Gerät eine Sonotrode angekoppelt wird, welche eine hakenartige Gestalt hat, mit welcher dann, den engen Platzverhältnissen angepasst, ganz gezielt die Schwingungsenergie über die Spitze dieser hakenartigen Vorrichtung an den gewünschten Ort getragen werden kann. Problematisch an derartigen Vorrichtungen ist es, dass die Umlenkung von Ultraschallschwingungen eine komplexe Angelegenheit ist, und entsprechend können derartige Vorrichtungen, wenn beispielsweise an der Ankopplungsstelle eine Ultraschallschwingung in axialer Richtung angelegt wird, nicht sicherstellen, dass auch an der unter einem Winkel zu dieser Ankopplungsstelle stehenden Spitze der Sonotrode eine ebenfalls nur entlang der Achse der Spitze verlaufende Ultraschallschwingung anliegt. Somit entstehen an der Spitze unter anderem auch laterale Schwingungen, die wiederum, beispielsweise insbesondere im medizinischen Bereich, äusserst ungewünscht sind, weil sie einerseits bei der Bearbeitung beim Patienten zu unangenehmen Schmerzen führen können, sondern auch weil sie das umliegende Gewebe gegebenenfalls nachhaltig schädigen können. Zudem ergibt sich dadurch ein ineffizienter Energieeintrag und also eine ungewünschte Verlängerung der Behandlungszeit.

Es gibt auf der anderen Seite Vorrichtungen, die ganz gezielt zum Zweck haben, Ultraschallschwingungen umzulenken. Dies geschieht dann über Biegeschwingungen von entsprechend spezifisch ausgebildeten Sonotroden, mit anderen Worten wird eine axiale Schwingung, die am Fuss respektive entlang der Befestigungsachse der Sonotrode angelegt wird, umgewandelt in eine Biegeschwingung in einem normalerweise zu diesem Zweck spezifisch gekrümmten Zwischenelement der Sonotrode, und dann am Ende der Sonotrode dieses eine Biegeschwingung ausführenden Kupplungselementes in eine Spitze überführt, welche geometrisch so angeordnet ist, dass an der Spitze gezielt eine axiale Ultraschallschwingung in diesem Spitzenelement anliegt. Vorrichtungen dieser Art, die dann üblicherweise ring- oder kreisförmige, die Biegeschwingung übernehmende Zwischenelemente aufweisen, sind beispielsweise aus der EP 0 594 541, der WO 2005/009256, der US 2010/0130867, oder aus der DE 20113692 bekannt.

Solche Vorrichtungen weisen den Nachteil auf, dass die gekrümmten, die Biegeschwingung durchführenden Zwischenelemente komplexe, und insbesondere stark belastete Elemente sind, entsprechend auch abnützungsanfällig sind, und die zudem viel Platz benötigen. Eine auf dem gleichen Prinzip beruhende Konstruktion ist aus der WO 2007/101362 bekannt, hier wird aber kein wirklich ringförmiges Biegeschwingungszwischenelement eingesetzt, sondern nur ein in Form eines Teilkreises ausgebildetes gekrümmtes Biegeschwingelement.

In der JP 63037923, US 2004/0112547, US 2010/0179654 und US 2006/0253050 werden Ultraschall-Schwingungen um einen bestimmten Winkel umgelenkt.

### DARSTELLUNG DER ERFINDUNG

Es ist entsprechend Aufgabe der vorliegenden Erfindung, eine verbesserte Sonotrode vorzuschlagen, insbesondere eine, welche in der Lage ist, ganz gezielt eine Umlenkung von entlang nur einer Achse oszillierenden Ultraschall-Schwingungen auf kleinem Raum um einen bestimmten Winkel umzulenken, d.h. so dass wiederum entlang der zweiten Achse im wesentlichen nur entlang dieser zweiten Achse oszillierende Ultraschall-Schwingungen resultieren.

Konkret geht es darum, eine Sonotrode zum Anschluss an einen Ultraschall-Schwingungsgeber vorzuschlagen mit einem sich entlang einer ersten Achse erstreckenden Anschlussabschnitt, der mit einem ersten Ende am Schwingungsgeber befestigt oder mit diesem verbunden ist, wobei der Anschlussabschnitt durch den Ultraschall-Schwingungsgeber im wesentlichen ausschließlich in eine Schwingung entlang der ersten Achse versetzt wird.

Die Aufgabe wird durch die Merkmale von Anspruch 1 erfüllt.

Insbesondere ist eine Sonotrode, wie sie vorliegend vorgeschlagen wird, dadurch gekennzeichnet, dass am freien, dem ersten Ende gegenüberliegenden Ende des Anschlussabschnittes der Sonotrode in einem gemeinsamen Kreuzungsbereich sowohl eine im wesentlichen zylindrische, erste Manschette angeordnet ist, welche sich entlang einer zweiten Achse erstreckt, als auch eine im wesentlichen zylindrische, zweite Manschette, welche sich entlang einer dritten Achse erstreckt, wobei die erste Achse mit der zweiten Achse einen ersten Winkel im Bereich von 100-140° einschließt, und wobei die erste Achse mit der dritten Achse einen zweiten Winkel im Bereich von 100-140° einschließt, und wobei die drei Achsen im wesentlichen in einer Sonotrodenebene angeordnet sind.

Üblicherweise sind dabei bevorzugtermassen die erste und die zweite Manschette kreiszylindrisch ausgebildet, sie können aber auch eine andere Querschnittsfläche aufweisen, beispielsweise eine ovale oder eine polygonale Querschnittsfläche. Der Anschlussabschnitt kann ebenfalls kreiszylindrisch ausgebildet sein, er kann aber auch eine andere Querschnittsfläche, beispielsweise eine rechteckige Querschnittsfläche oder eine andere polygonale oder eine ovale Querschnittsfläche aufweisen.

Dabei ist gewissermassen einer der Kernpunkte der Erfindung darin zu sehen, dass im Gegensatz zu den komplizierten Umlenkungsvorrichtungen nach dem Stand der Technik, welche in der Regel Biegeschwingungen zur Umlenkung von einer ersten Richtung in eine zweite Richtung einsetzen (was dann entsprechende grosse Elemente voraussetzt, welche diese Biegeschwingung übernehmen können), so vorgegangen wird, dass gewissermassen eine Y-förmige Struktur eingesetzt wird, welche hinsichtlich Massenverteilung und geometrischer Ausgestaltung so ausgelegt ist, dass entlang aller Schenkel jeweils im wesentlichen nur Ultraschall-Schwingungen entlang der Achse der jeweiligen Schenkel vorliegen. Mit anderen Worten wird eine balancierte Struktur vorgeschlagen, welche auf kleinem Raum eine gezielte Umlenkung der Ultraschall-Schwingungen in axialer Richtung ermöglicht. Vorzugsweise wird dabei in den Schenkeln, insbesondere an der Spitze der Manschette, im Falle einer hohlzylindrischen Ausbildung derselben an der umlaufenden Kante also an der Spitze der jeweiligen Manschette im Bereich der vorderen Öffnung, der Anteil an Schwingungen, die nicht axial sind (d.h. Biegeschwingungen im Schenkel oder um den Kreuzungspunkt) nicht mehr als 15%, vorzugsweise nicht mehr als 10%, und insbesondere nicht mehr als 5% der gesamten Ultraschall-Schwingungsenergie ausmachen. D.h. der Grossteil der Schwingungsenergie, d.h. mehr als 85%, vorzugsweise mehr als 90%, besonders vorzugsweise mehr als 95% und ganz besonders bevorzugt wenigstens 98% der Ultraschalllschwingungsenergie in jedem der Schenkel, , insbesondere an der Spitze der Manschette, im Falle einer hohlzylindrischen Ausbildung derselben an der umlaufenden Kante also an der Spitze der jeweiligen Manschette im Bereich der vorderen Öffnung, liegt als axiale Schwingung an.

Gemäß einer ersten bevorzugten Ausführungsform ist eine solche dadurch gekennzeichnet, dass das Verhältnis von Außendurchmesser zur sich entlang der zweiten Achse respektive der dritten Achse erstreckenden Länge der beiden Manschetten im Bereich von 1:2-1:10, vorzugsweise im Bereich von 1:3-1:6 liegt. Es zeigt sich, dass bei einer derartigen Dimensionierung der beiden Manschetten, welche gewissermassen die axiale Schwingung entlang des Anschlussabschnittes weitgehend symmetrisch in zwei unterschiedliche Raumrichtungen aufteilen, wobei wiederum in diesen beiden unterschiedlichen Raumrichtungen axiale Schwingungen vorliegen, diese Verhältnisse die oben genannte Massenverteilung gut sicherstellen können. Es zeigt sich insbesondere, dass bei Verhältnissen unterhalb von 1:2 ein unbefriedigendes Schwingungsverhalten resultiert, und dass bei Verhältnissen oberhalb von 1:10 die entsprechende Manschette zu lang ist und zu flattern beginnt. Bevorzugtermassen ist dabei der Anschlussbereich mit einer ähnlichen Länge ausgebildet wie die beiden Manschetten, um die genannte Symmetrie weiterhin zu optimieren. Mögliche detailliertere mögliche Dimensionierungen sind weiter unten ausgeführt.

Bevorzugtermassen ist dabei, um die genannte Symmetrie und optimale Verteilung der Schwingungen sicherzustellen, der erste Winkel γ und/oder der zweite Winkel β im Bereich von 110-130°, vorzugsweise im Bereich von 115-125°, insbesondere vorzugsweise im Bereich von 118-122°. Insbesondere bevorzugt sind die beiden Winkel im wesentlichen 120° so dass ein symmetrisches Element mit einer dreizähligen Drehachse vorliegt. Gemäß einer weiteren bevorzugten Ausführungsform sind der erste Winkel γ Und der zweite Winkel β bis auf ein abweichen von nicht mehr als 5°, vorzugsweise von nicht mehr als 2° gleich. Ganz besonders bevorzugt sind die beiden Winkel im wesentlichen genau gleich.

Gemäß einer weiteren bevorzugten Ausführungsform schiessen die zweite Achse und die dritte Achse einen dritten Winkel α ein, welcher im Bereich von 100-140°, insbesondere im Bereich von 110-130°, insbesondere bevorzugt im Bereich von 105-125° liegt.

Bevorzugtermassen sind sowohl die erste Manschette als auch die zweite Manschette als Kreiszylinder mit einer kreiszylindrischen Außenfläche ausgebildet. Weiterhin bevorzugt ist auch der Anschlussbereich als Kreiszylinder ausgebildet.

Weiterhin bevorzugtermassen ist der Außendurchmesser D der ersten Manschette größer als der Außendurchmesser d der zweiten Manschette. So ist es möglich, die beiden Manschetten für unterschiedliche Ausnehmungen einzusetzen, es entsteht entsprechend ein Werkzeug, welches durch seine symmetrische Ausgestaltung eine optimale Umlenkung der Ultraschall-Schwingungen in zwei Richtungen und entlang der Achsen ohne laterale Schwingungen gewährleistet, wo aber gleichzeitig beide Manschetten für die eigentlichen Arbeitsschritte eingesetzt werden können, und also nicht einfach nur eine der Manschetten zum Zwecke der Balancierung der Umlenkung der Ultraschall-Schwingungen vorgesehen wird. Zudem können die beiden Manschetten für unterschiedliche Anwendungen gezielt eingesetzt werden. Dabei ist der große Durchmesser vorzugsweise 1.1-3 mal, insbesondere vorzugsweise 1.2 - 2 mal größer als der kleine Durchmesser.

Gemäß einer weiteren bevorzugten Ausführungsform ist das Verhältnis von Außendurchmesser d, D zur sich entlang der zweiten Achse respektive der dritten Achse erstreckenden Länge 1, L der beiden Manschetten im wesentlichen gleich, dies wiederum, um eine möglichst effiziente Massenverteilung sicherzustellen.

Eine weitere besonders bevorzugte Ausführungsform ist dadurch gekennzeichnet, dass die erste Manschette und/oder die zweite Manschette, vorzugsweise beide, eine zentrale axiale zylindrische Ausnehmung aufweisen, welche zum jeweiligen freien Ende unter Ausbildung einer vorderen Öffnung offen liegen. Mit anderen Worten sind die beiden Manschetten als Hohlzylinder ausgebildet, sie können aber auch als Vollzylinder ausgebildet sein. So kann eine derartige Sonotrode beispielsweise in einem Verfahren eingesetzt werden, wie es in der WO 2009/141252 beschrieben ist. Es ist auch möglich, eine der Manschetten als Hohlzylinder und eine als Vollzylinder auszubilden. Wird eine (oder beide) Manschette als Vollzylinder ausgebildet, so übernimmt sie die Funktion des Führungsstiftes im Verfahren gemäß der WO 2009/141252, wird eine (oder beide) Manschette als Hohlzylinder ausgebildet, und dies ist die bevorzugte Variante, so wird sie als Manschette im Sinne des Verfahrens gemäß der WO 2009/141252 eingesetzt. Ist jeweils ein Hohlzylinder ausgebildet, so verfügt der damit gebildete zylindrische Innenraum vorzugsweise über eine konstante Querschnittsfläche über die Länge. Es ist auch möglich, nur eine der Manschetten als Hohlzylinder auszubilden und die andere Manschette, z.B. als Vollzylinder, so kurz und damit platzsparend wie möglich aber gerade so geometrisch und massenmässig ausgestaltet, dass sie das Schwingungsverhalten in der hohlen Manschette so tariert, dass dort nur axiale Schwingungen anliegen.

Dabei ist bevorzugtermassen die im Bereich dieser vorderen Öffnung der jeweiligen Manschette dadurch gebildete umlaufende Kante konisch zur Spitze hin zulaufend ausgebildet, d.h. an der innenliegenden Kante, deren Durchmesser im Innendurchmesser des Hohlzylinder entspricht, liegt eine bevorzugtermassen scharfe Kante vor. Konisch zulaufend kann dabei im Sinne einer gestuften Form, einer Form in einer axialen Schnittdarstellung entlang einer Geraden, oder aber auch gewissermassen konkav konisch zulaufend umfassen. Dies wiederum, um die Verwendung im Zusammenhang mit einem Verfahren gemäß der WO 2009/141252 zu optimieren, mit anderen Worten um eine um einen Führungsstift im Verfahren angelegte durch Ultraschall verflüssigbare Hülse möglichst optimal in die poröse Wandstruktur der zu ameliorierenden Ausnehmung auszutragen.

Gemäß einer weiteren bevorzugten Ausführungsform ist die den Hohlzylinder innenseitig definierende Ausnehmung eine Durchgangsbohrung mit über die gesamte Länge konstantem Innendurchmesser, welche an der jeweiligen Stelle des Kreuzungsbereichs zudem über eine jeweilige rückseitige Öffnung zugänglich ist, so dass ein Führungsstift mit entsprechendem Außendurchmesser sowohl von vorne als auch von hinten eingeschoben oder ganz hindurchgeschoben werden kann.

Eine weitere bevorzugte Ausführungsform ist dadurch gekennzeichnet, dass sowohl Anschlussbereich als auch beide Manschetten als röhrenförmige Hohlzylinder ausgebildet sind, welche im Kreuzungsbereich zusammenlaufen (beispielsweise wenn die Sonotrode einstückig ausgebildet ist) respektive miteinander verbunden sind (beispielsweise wenn die einzelnen Elemente miteinander verschraubt sind). Dies bevorzugtermassen so, dass durch beide Manschetten von jeweils beiden Seiten zugängliche Durchgangsöffnungen vorhanden sind und am freien Ende des Anschlussbereichs eine vordere Öffnung vorhanden ist. Diese vordere Öffnung kann aber auch, beispielsweise aus hygienischen Gründen, durch einen Zapfen oder ähnliches geschlossen sein. Wird auch der Anschlussbereich als Hohlzylinder ausgebildet, so ist dieser vorzugsweise hinsichtlich Wandstärke und/oder Außendurchmesser und/oder Länge ähnlich dimensioniert wie eine oder beide der Manschetten.

Eine weitere bevorzugte Ausführungsform zeichnet sich dadurch aus, dass die erste Manschette und die zweite Manschette eine, insbesondere bezüglich einer durch die erste Achse und eine Ebenennormale zur Sonotrodenebene aufgespannte Spiegelebene symmetrische Massenverteilung aufweisen. Bevorzugtermassen wird dies beispielsweise gewährleistet, indem, zur Kompensation von Unterschieden in der Länge und/oder im Durchmesser und/oder im Innendurchmesser oder aber auch im Material der beiden Manschetten an der Sonotrode im Kreuzungsbereich Materialverdickungen und/oder Materialausnehmungen ausgebildet sind. Solche Verdickungen können auch zur Stabilisierung eingesetzt werden, und/oder sie können auch im Bereich des freien Endes des Anschlussbereichs vorgesehen sein. Der Kreuzungsbereich kann auch gewissermassen als separater Block ausgebildet sein, an welchem der Anschlussbereich und die beiden Manschetten angeformt sind, oder in welchen die beiden Manschetten eingeschraubt sind. Der Kreuzungsbereich kann somit beispielsweise für die Befestigung von unterschiedlichen Manschetten für unterschiedliche Anwendungsbereiche ausgelegt sein, der Kreuzungsbereich kann zudem durch seine spezifische Massenverteilung dazu eingesetzt werden, das Schwingungsverhalten der Sonotrode gewissermassen zu tarieren, so dass am Ende als einfache Hohlzylinder ausgestaltete unterschiedliche Manschetten eingeschraubt werden können, welche ihrerseits nicht mehr spezifisch für die optimale Schwingungsverteilung ausgelegt sind, da diese Funktion durch die Ausgestaltung des Kreuzungsbereichs übernommen wird.

Gemäß einer weiteren bevorzugten Ausführungsform besteht die Sonotrode aus einem, gegebenenfalls beschichteten, metallischen Werkstoff. Dieser Werkstoff ist vorzugsweise ausgewählt aus der Gruppe bestehend aus: Aluminium, Eisen, Titan, Stahl sowie diese in der Hauptsache enthaltende oder aus diesen weitgehend bestehende Legierungen. Generell ist es möglich, wie bereits oben erwähnt, die Sonotrode einstückig auszubilden, beispielsweise aus einem der oben angegebenen Materialien. In diesem Fall ist die Sonotrode beispielsweise aus einem Werkblock herausgefräst. Alternativ ist es möglich, wie ebenfalls bereits oben erwähnt, die Sonotrode aus miteinander verbundenen Einzelelementen auszubilden. Die einzelnen individuellen und mit einander verbundenen Elemente können dabei z.B. die beiden Manschetten, ein Kreuzungsteil und ein Anschlussabschnitt sein. Die Verbindung kann bevorzugtermassen durch Formschluss und/oder Kraftschluss erfolgen. Auch ein Stoffschluss ist alternativ oder zusätzlich möglich, dieser sollte aber mit den Ultraschall-Eigenschaften nicht negativ wechselwirken, was bei einigen solchen Verbindungsweisen der Fall ist. So ist es z.B. möglich die Sonotrode z.B. aus ineinander geschraubten, gesteckten, verpressten oder vernieteten Elementen auszubilden, indem der Anschlussabschnitt und/oder der Kreuzungsbereich und/oder die erste Manschette und/oder die zweite Manschette miteinander verschraubt/gesteckt/verpresst/vernietet werden. Beispielsweise ist es möglich, einen Kreuzungsbereich in Form eines Blockes mit drei unter den entsprechenden Winkeln abzweigenden Innengewinden auszubilden, so dass anschließend als einfache Hohlzylinder mit Außengewinde im entsprechenden Bereich ausgebildete Anschlussabschnitte respektive Manschetten eingeschraubt werden können. Bevorzugtermassen ist die Sonotrode frei von Schweißstellen, da diese das Schwingungsverhalten negativ beeinflussen können. Wie erläutert können Beschichtungen eingesetzt werden, so insbesondere Beschichtungen, welche z.B. die Gleiteigenschaften, die Hygieneeigenschaften, und/oder die Schwingungsübertragungseigenschaften beeinflussen. Möglich sind beispielsweise, insbesondere im Bereich der Manschetten, Beschichtungen aus Kunststoff wie beispielsweise PTFE.

Hinsichtlich Dimensionierung hat es sich, insbesondere zur Amelioration von Bohrungen beispielsweise im Möbelbereich, oder generell bei der Verbindung von porösen Werkstoffen wie z.B. Holz, Schäumen (Metallschäume, Kunststoffschäume etc), Verbundwerkstoffen, oder aber auch im medizinischen Bereich beispielsweise bei Bohrungen im Knochen oder porösem Gewebe, als vorteilhaft erwiesen, wenn beide Manschetten eine Länge im Bereich von 5-50 mm, vorzugsweise im Bereich von 10-25 mm aufweisen und einen Durchmesser im Bereich von 2-15 mm, vorzugsweise im Bereich von 2.5-10 mm, insbesondere vorzugsweise im Bereich von 2.5-7 mm. Wie bereits oben erwähnt ist es von Vorteil, wenn beispielsweise die Dimensionierung der beiden Manschetten hinsichtlich Außendurchmesser spezifisch ausgelegt wird für zwei Standard-Bohrungsdurchmesser, denkbar ist beispielsweise, dass eine erste Manschette einen Außendurchmesser von 4.3 mm und eine zweite Manschette einen Außendurchmesser von 3.5 mm aufweist.

Bevorzugte Paare von Manschetten sind dimensioniert wie folgt:
- Kombination von zwei kleinen Durchmessern 2.8mm (Aussendurchmesser erste Manschette) und 3.5mm (Aussendurchmesser zweite Manschette) in einer Sonotrode;
- Kombination von zwei grossen Durchmessern 4.3mm (Aussendurchmesser erste Manschette) und 5.3m (Aussendurchmesser zweite Manschette).

Prinzipiell lassen sich aber auch andere Kombinationen umsetzen. Um die Massenverteilung zu kompensieren ist es dann möglich, die Länge etwas anzupassen, d.h. die Manschette mit dünnerem Außendurchmesser etwas länger auszubilden als jene mit dickerem Außendurchmesser. Für die oben angegebenen beiden Maße ist es beispielsweise möglich, die Manschette mit dem Außendurchmesser von 4.3 mm mit einer Länge von ca. 15 mm auszugestalten und jene mit Außendurchmesser von 3.5 mm mit einer Länge von 16 mm. Die spezifische Anpassung und Austarierung des Schwingungsverhaltens kann üblicherweise weitgehend durch eine derartige Längenanpassung der Manschetten erreicht werden, so dass die Optimierung des Schwingungsverhaltens relativ einfach durch Versuche oder Simulationen ermittelt werden kann. Eine zusätzliche Anpassung kann durch die Ausgestaltung des Kreuzungsbereichs, beispielsweise durch Materialverdickungen oder -ausnehmungen, bewirkt werden.

Die entsprechende Durchgangsöffnung durch zwei derartig unterschiedliche Manschetten kann dabei gleich ausgebildet sein hinsichtlich Innendurchmesser, um den gleichen Führungsstift einsetzen zu können. Es ist aber auch möglich, die Durchgangsöffnungen durch die jeweilige Manschette angepasst unterschiedlich auszugestalten (beispielsweise um eine optimale Wandstärke beizubehalten), wobei dann bei Verwendung unterschiedlicher Manschetten auch unterschiedliche Führungsstifte eingesetzt werden müssen.

Bevorzugtermassen ist am ersten Ende des Anschlussabschnitts eine Schnittstelle zur Befestigung am Ultraschall-Schwingungsgeber vorgesehen, vorzugsweise in Form eines formschlüssigen und/oder kraftschlüssigen Kupplungsbereiches, insbesondere bevorzugt in Form eines Gewindes, eines Flansches, einer Nut oder eines Bajonettverschlusses.

Des weiteren betrifft die vorliegende Erfindung ein Sonotrodenwerkzeug mit einem Ultraschall-Schwingungsgeber und einer Sonotrode wie oben detailliert, wobei bevorzugtermassen der Schwingungsgeber einen als Handgriff ausgebildeten Konverter umfasst sowie einen Booster, wobei die Sonotrode am Booster befestigt ist oder mit diesem einstückig ausgebildet ist. Ein entsprechendes Werkzeug verfügt typischerweise zudem über einen Elektroanschluss und über eine Steuerung, mit welcher die angelegte Schwingung eingestellt werden kann. Üblicherweise werden Schwingungen im Bereich von 20-120 kHz angelegt, vorzugsweise im Bereich von 30-80 kHz, insbesondere bevorzugt im Bereich von 50-80 kHz.

Ein solches Sonotrodenwerkzeug ist bevorzugtermassen dadurch gekennzeichnet, dass es als Handwerkzeug für den medizinischen Bereich, insbesondere für den Implantatbereich ausgelegt ist, insbesondere für den dentalen Bereich.

Des weiteren betrifft die vorliegende Erfindung die Verwendung einer solchen Sonotrode oder eines derartigen Sonotrodenwerkzeugs zur Amelioration einer Ausnehmung,, namentlich im Rahmen eines Verfahrens, wie es beispielsweise in der WO 2009/141252 ausführlich beschrieben ist. Es kann sich dabei um ein medizinisches Verfahren handeln oder um ein nicht-medizinisches Verfahren. Hinsichtlich Verfahren sei entsprechend der Offenbarungsgehalt der WO 2009/141252 ausdrücklich in diesen Offenbarungsgehalt mit eingeschlossen. Es geht mit anderen Worten um ein Verfahren zur Amelioration einer Ausnehmung in einem porösen, löchrigen und durch die Ausnehmung freigelegte Hohlräume aufweisenden Material, wobei eine der beiden zylindrischen Manschette mit zylindrischer Mantelfläche mit einem Aussendurchmesser und mit einer zentralen Ausnehmung zur Aufnahme eines Führungsstiftes, eingesetzt wird, wobei der Führungsstift dazu vorgesehen ist, vor dem Anlegen von mechanischer Energie im wesentlichen bis auf den Boden der Ausnehmung eingeführt zu sein, wobei der Führungsstift im Bereich seines dem Boden der Ausnehmung zugewandten Endes von einer Ameliorationshülse aus einem mit mechanischer Energie verflüssigbaren Material umschlossen wird, wobei die zylindrische Mantelfläche der Ameliorationshülse im wesentlichen den gleichen Aussendurchmesser aufweist wie die Manschette, und wobei der Führungsstift in der zentralen Ausnehmung derart verschieblich aufgenommen ist, dass die Manschette bei Anlegen von mechanischer Energie relativ zum Führungsstift in Richtung zum Boden der Ausnehmung unter Verflüssigung und seitlicher und/oder longitudinaler Verdrängung des Materials der Ameliorationshülse verschoben werden kann.

Weitere Ausführungsformen sind in den abhängigen Ansprüchen angegeben.

### KURZE BESCHREIBUNG DER ZEICHNUNGEN

Bevorzugte Ausführungsformen der Erfindung werden im Folgenden anhand der Zeichnungen beschrieben, die lediglich zur Erläuterung dienen und nicht einschränkend auszulegen sind. In den Zeichnungen zeigen:
- Fig. 1: in einer seitlichen Ansicht ein Sonotrodenwerkzeug, bei welchem die Sonotrode teilweise axial geschnitten dargestellt ist und in der Papierebene liegt, und wobei der Führungsstift vorne aus der ersten Manschette herausragt;
- Fig. 2: in a) in einer seitlichen Ansicht die Sonotrode gemäß Figur 1, wobei der Führungsstift rückseitig aus der ersten Manschette herausragt, und in b) ein Sonotrodenwerkzeug gemäß Figur 1 mit einem langen Führungsstift, der beidseitig aus der ersten Manschette herausragt;
- Fig. 3: schematisch in einer perspektivischen Ansicht ein in eine Mundöffnung eingeführtes Sonotrodenwerkzeug oberhalb einer vorbereiteten Bohrung im Unterkiefer;
- Fig. 4: eine Sonotrode in einer Darstellung entlang der Sonotrodenebene auf die erste Manschette;
- Fig. 5: eine Sonotrode in einer perspektivische Darstellung; und
- Fig. 6: mögliche Bemassungen einer Sonotrode.

### BESCHREIBUNG BEVORZUGTER AUSFÜHRUNGSFORMEN

Insbesondere für den medizinischen Bereich, namentlich für den Implantatbereich und ganz besonders für den Dentalimplantatbereich ist beispielsweise aus der WO 2009/141252 ein Verfahren zur Amelioration von Ausnehmungen in porösen Materialien bekannt. Dabei wird eine im wesentlichen zylindrische Hülse aus einem unter Ultraschall verflüssigenden Material dazu eingesetzt, gewissermassen die Porosität der Wandungsbereiche der Ausnehmung mit diesem Material unter Einwirkung von Ultraschall verflüssigend auszufüllen. Dies geschieht so, dass ein Führungsstift, der in einer mit einer entsprechenden axialen Ausnehmung versehenden Manschette geführt ist, in eine bereits vorgesehene Bohrung als Führungselement eingeführt wird, und anschliessend die gleitend darauf gelagerte Manschette nach unten in Richtung des Bodens der Ausnehmung geschoben wird. Um den Führungsstift herum ist im Spitzenbereich ein Hohlzylinder aus einem verflüssigbaren Material vorgesehen, und dieser wird durch die Manschette, die einen Aussenumfang aufweist, der im wesentlichen dem Innendurchmesser der zu ameliorierenden Bohrung entspricht, durch das nach unten verschiebende und gleichzeitige Verflüssigen dieser Materialhülse nach aussen in die Porosität verdrängt und füllt entsprechend Hohlräume im Material aus.

Im Zusammenhang mit einem solchen Verfahren ist es von Vorteil, wenn nur an der Manschette, die über den Führungsstift geführt wird, eine Ultraschallschwingung anliegt, damit gewissermassen das zu verflüssigende Material jeweils immer nur dort, wo es in Kontakt mit dieser Manschette ist, verflüssigt wird. Zudem sollten an dieser Manschette laterale Ultraschallschwingungen vermieden werden, d.h. die Ultraschallschwingungen sollten entlang der Achse dieser Manschette verlaufen. Da gerade im Dentalbereich die Platzverhältnisse beengt sind, besteht entsprechend ein Bedarf nach Vorrichtungen, welche dazu in der Lage sind, die Ultraschallschwingung eines Handgeräts in eine Richtung umzulenken, so dass sie dann optimal bei einer Bearbeitung einer Bohrung eingesetzt werden kann. Eine solche Vorrichtung soll in der Folge beschrieben werden.

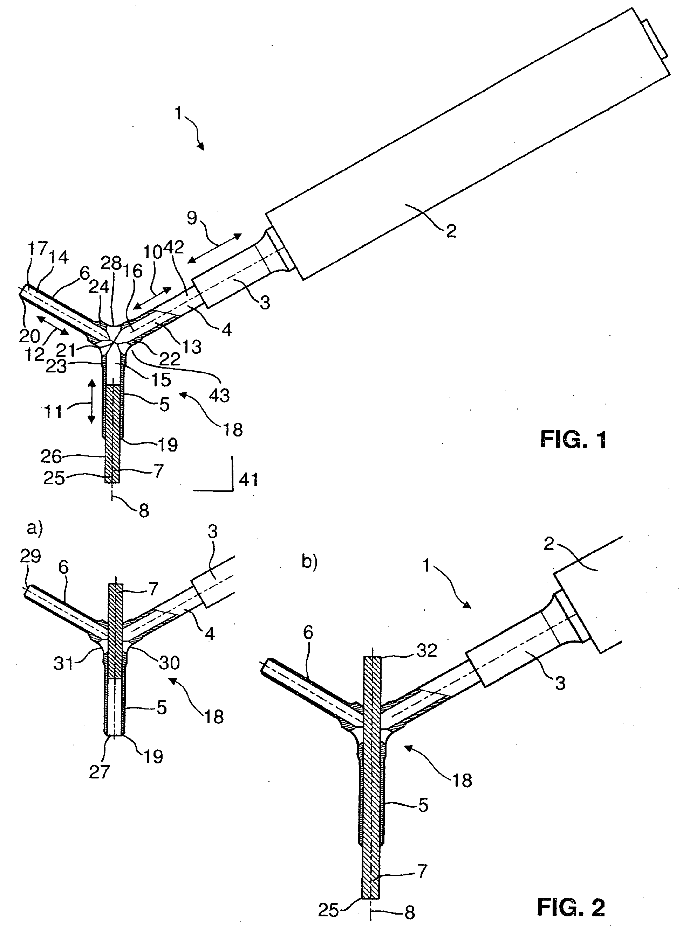

Figur 1 zeigt in einer seitlichen Ansicht ein Sonotrodenwerkzeug 1 mit einem Handgriff in Form eines Konverters 2, in welchem der eigentliche Ultraschall erzeugt wird. Der Ultraschall wird mit einer Frequenz im Bereich von beispielsweise 30 bis 70 kHz angelegt und die Amplitude beträgt ca. 1-5 Mikrometer, die Ultraschallschwingung verläuft in einer axialen Richtung, wie sie schematisch mit dem Pfeil 9 angedeutet ist. Die Ultraschallschwingung wird zunächst auf einen sogenannten Booster 3 übertragen, in welchem die Amplituden verstärkt werden, so dass die Ultraschallschwingungen anschliessend im Bereich der eigentlichen Sonotrode 18 eine Amplitude im Bereich zwischen 5-25 Mikrometer aufweisen.

An diesem Booster 3 ist das eigentliche Sonotrodenelement 18 befestigt. Dies beispielsweise über eine Kupplungsstelle in Form eines Gewindes, es sind aber auch andere Kupplungsmöglichkeiten denkbar. Die eigentliche Sonotrode 18 verfügt über einen zylindrisch ausgebildeten Anschlussabschnitt 4, der in diesem Fall als Hohlzylinder ausgebildet ist, d.h. er verfügt über einen entlang der Achse 16 verlaufenden inneren Hohlraum 13. Dieser Hohlraum ist nach vorne hin offen, d.h. es gibt eine vordere Öffnung 31.

Am freien vorderen Ende dieses Anschlussabschnittes der Sonotrode 18 ist ein Kreuzungsbereich 36 angeordnet. Bei diesem Kreuzungsbereich 36 verzweigt sich die Sonotrode 18 in zwei in unterschiedliche Richtungen zeigende Manschetten, eine erste Manschette 5 mit einem grösseren Aussendurchmesser und eine zweite Manschette 6 mit einem kleineren Aussendurchmesser. Die beiden Abschnitte 5 und 6 verzweigen sich symmetrisch, d.h. die Achse 8 der ersten Manschette 5 und die Achse 17 der zweiten Manschette 6 bilden untereinander einen Winkel von 120 Grad, diese Achsen wiederum ihrerseits einen gleichen Winkel mit der bereits genannten Achse 16 des Anschlussabschnittes 4, und die einzelnen Schenkel dieses Dreibeins liegen alle in einer gemeinsamen Ebene, die schematisch durch das Bezugszeichen 41 angegeben ist und die in dieser Darstellung in der Papierebene liegt.

Sowohl die erste Manschette 5 als auch die zweite Manschette 6 sind als Hohlzylinder ausgebildet, d.h. sie umschliessen jeweils einen Hohlraum 15 resp. 14. Dieser Hohlraum ist dabei jeweils als Durchgangsöffnung durch den gesamten Sonotrodenbereich ausgestaltet, d.h. am vorderen Ende der ersten Manschette gibt es eine vordere Öffnung 27 und rückseitig eine hintere Öffnung 28, und bei der zweiten Manschette 6 gibt es ebenfalls eine vordere Öffnung 29 und eine rückseitige Öffnung 30 (vgl. auch Figur 2). In diese jeweiligen Durchführungsöffnungen, die einen über die gesamte Länge konstanten Innendurchmesser aufweisen, kann ein Führungsstift 7 verschieblich eingeschoben werden. Wird über den Booster 3 eine Ultraschwingung in axialer Richtung wie gemäss Pfeil 9 angelegt, so resultiert dies in einer ebenfalls axialen Ultraschallschwingung im Anschlussabschnitt 4, wie dies schematisch durch den Pfeil 10 angedeutet ist. Durch die symmetrische Ausgestaltung der beiden Schenkel 5 resp. 6 wird diese axiale Schwingung verteilt in zwei wiederum axiale Schwingungen in den jeweiligen Manschetten, wie dies durch die Pfeile 11 und 12 dargestellt ist. Dies ganz im Sinne der Symmetrieüberlegungen, wie sie beispielsweise aus dem Schwingungsverhalten von Molekülen mit ähnlicher Symmetriegruppe bekannt sind. Ist die Sonotrode 18 vollständig symmetrisch ausgebildet, d.h. sind die beiden Manschetten 5 und 6 mit gleicher Länge, gleicher Wandstärke und aus gleichem Material und mit gleichem Durchmesser ausgebildet, so verteilt sich die Schwingung jeweils axial gleichmässig auf die beiden Manschetten 5 und 6 und verläuft auch in diesen Manschetten, insbesondere wenn die jeweiligen Schenkelwinkel von 120 Grad einschliessen, entlang der jeweiligen Achse.

Da nun aber gleichzeitig der Zweck erreicht werden soll, dass die beiden Schenkel der Manschetten 5, 6 für unterschiedliche Anwendungen eingesetzt werden können, d.h. mit unterschiedlichem Aussendurchmesser für Bohrungen mit unterschiedlichem Durchmesser eingesetzt werden können, ist diese vollständige Symmetrie ganz gezielt hier nicht gegeben, dieser Tatsache wird aber zur Sicherstellung einer wirklich reinen axialen Schwingung im jeweiligen Schenkel Rechnung getragen, indem einerseits der Manschettenabschnitt 6 mit dem geringeren Durchmesser etwas länger ausgebildet ist, als der kürzere Manschettenabschnitt 5 mit dem grösseren Durchmesser, und indem zusätzlich Materialverdickungen 24 am Schenkel 6 in Kreuzungsbereich vorgesehen werden, und korrespondierende Materialverdickungen im Bereich 23 am Element 5. So kann das Schwingungsverhalten austariert werden und sichergestellt werden, dass tatsächlich nur die mit den Pfeilen in Figur 1 dargestellten axialen Schwingungen an den einzelnen Schenkeln anliegen.

Zur Durchführung des Eingangs erwähnten Verfahrens gemäss der WO 2009/141252 ist zudem die umlaufende Kante der jeweiligen Manschette 5, 6 an der Spitze, d.h. die distale Kante 19, konisch zulaufend ausgebildet, um eine möglichst konzentriert seitliche Verdrängung einer aus einem verflüssigbaren Material bestehenden Materialhülse, die auf einen Führungsstift 7 augeschoben ist, sicherzustellen.

Figur 2 zeigt, wie der Führungsstift 7 durch die zentrale Durchgangsöffnung in der Manschette 5 hindurch geschoben werden kann, in Figur 2a ist sie nach hinten hinaus geschoben, in Figur 2b ist ein langer Führungsstift vorgesehen, welcher sich durch die gesamte Durchgangsöffnung erstreckt und welcher mit anderen Worten mit seinem rückseitigen Ende 32 nach hinten hinausragt. Führungsstifte 7 können den Bedürfnissen entsprechend in unterschiedlicher Länge vorgesehen werden und können beispielsweise aus einem Kunststoff bestehen.

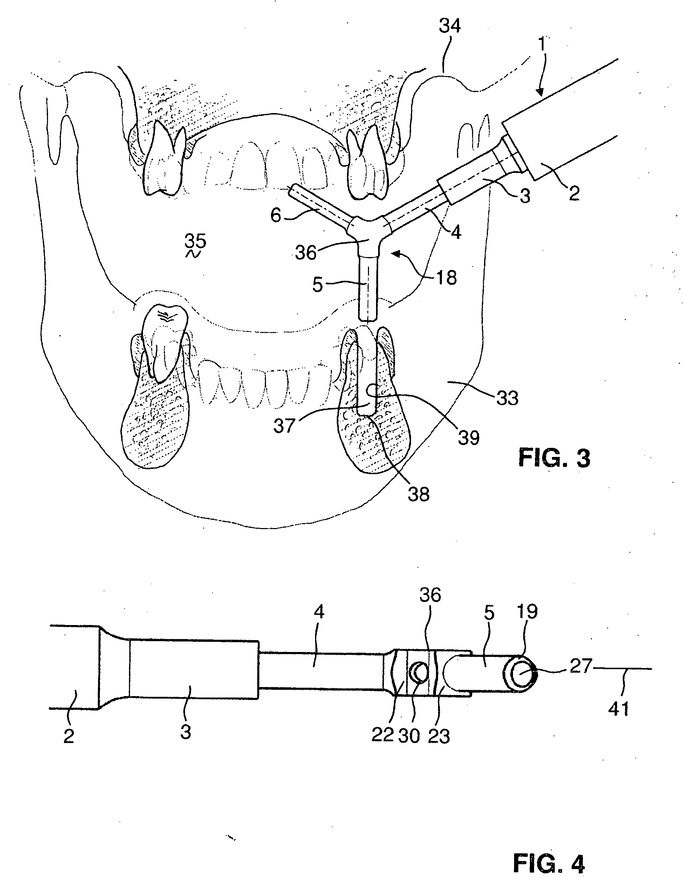

Figur 3 zeigt in schematischer Darstellung den Einsatz eines solchen Sonotrodenwerkzeugs im Dentalbereich, d.h. wo in einem Unterkiefer 33 eine vorbereitete Bohrung 37 vorgesehen ist, die über einen seitlichen umlaufenden porösen Wandbereich 39 verfügt. Bei einem Verfahren gemäss der WO 2009/141252 wird nun ein (hier nicht dargestellter) Führungsstift in die Bohrung eingeschoben, und am in die Bohrung hineinragenden Ende davon ist eine umlaufende Materialhülse aus unter Einwirkung der Schwingungen sich verflüssigendem Material vorgesehen. Die Manschette 5 wird nun in einer Abwärtsbewegung in Figur 3 sukzessive in die Bohrung hineinverschoben, so dass sich die hohlzylindrische Materialhülse im Kontaktbereich mit dem distalen Ende der Manschette sukksessive von oben nach unten verflüssigt und seitlich in den porösen Bereich 39 verdrängt wird, während der Führungsstift stationär bleibt.

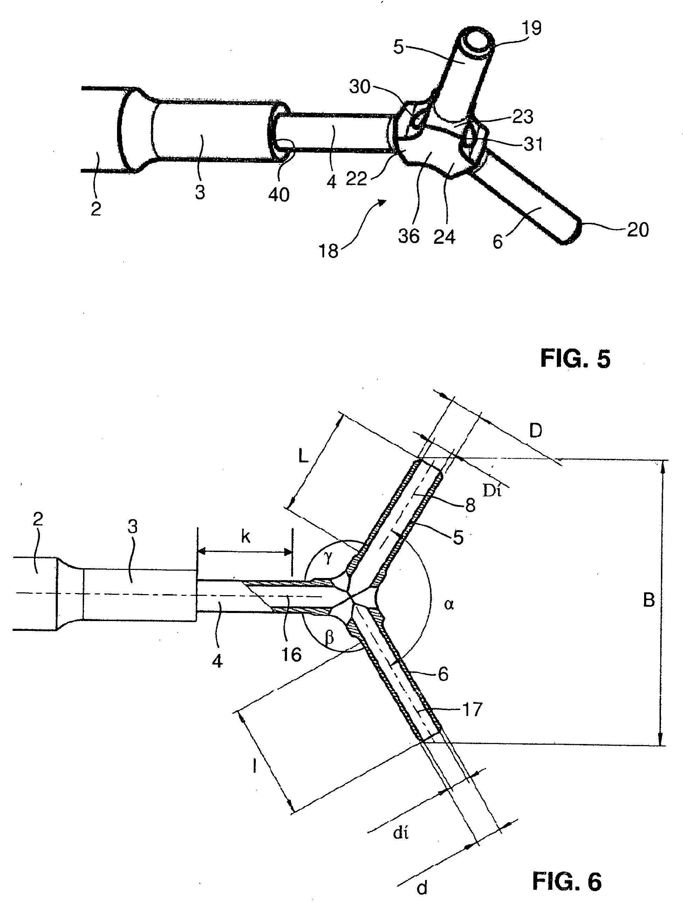

Die Figuren 4 und 5 zeigen perspektivische Ansichten einer solchen Sonotrode, hier wird erkennbar, wie der Kreuzungsbereich 36 in Form eines Blockes ausgebildet sein kann, dessen Gestaltung insbesondere auch an die Schwingungsverhältnisse angepasst sein kann. Figur 6 zeigt eine mögliche Dimensionierung und Ausgestaltung einer solchen Sonotrode, so liegt beispielsweise eine mögliche Länge L der ersten Manschette 5 im Bereich von 10-20 mm, z. B. ca. 15 mm, der Aussendurchmesser D ist beispielsweise 4,3 mm und der Innendurchmesser Dᵢ 3 mm, so dass hier ein Führungsstift 7 mit einem Aussendurchmesser von 3 mm eingesetzt werden könnte. Entsprechend käme dann eine Materialhülse aus verflüssigbarem Material mit einem Innendurchmesser von 3 mm und einem Außendurchmesser von 4.3 mm zur Anwendung.

Die zweite Manschette 6 verfügt ihrerseits über eine, wie oben beschrieben zur Austarierung der Schwingungen etwas länger ausgebildete Länge 1 im Bereich von 10-20 mm, hier konkret ca. 16 mm, der Aussendurchmesser d beträgt 3,5 mm und der Innendurchmesser di 2,5 mm. So wird, wenn diese Manschette eingesetzt wird für eine kleinere Bohrung mit einem Außendurchmesser von 3.5 mm dann ein Führungsstift 7 mit einem Aussendurchmesser von 2,5 mm Einsatz finden. Entsprechend käme dann eine Materialhülse aus verflüssigbarem Material mit einem Innendurchmesser von 2.5 mm und einem Außendurchmesser von 3.5 mm zur Anwendung.

Wie auch bereich in den vorherigen Figuren dargestellt ist auch der Anschlussabschnitt 4 als Hohlzylinder ausgebildet, d.h. er umschliesst einen Hohlraum. Dieser Abschnitt verfügt typischerweise über eine Länge k die ungefähr der Länge der einzelnen Manschetten entspricht. Im hier angegebenen konkreten Fall verfügt dieser Abschnitt über eine Länge k von ca. 15 mm. Die drei Schenkel schliessen jeweils Winkel α, β, γ ein, diese Winkel sind im hier dargestellten konkreten Beispiel alle 120 Grad.

**Bezugszeichenliste**

| | | | |
|---|---|---|---|
| 1 | Sonotrodenwerkzeug | | Bereich des |
| 2 | Konverter | | Kreuzungsbereichs an 5 |
| 3 | Booster | 24 | Materialverdickungen im |
| 4 | Anschlussabschnitt von 18 | | Bereich des |
| 5 | erster Arbeitsabschnitt von | | Kreuzungsbereichs an 6 |
| | 18, erste Manschette mit | 25 | freies distales Ende von 7 |
| | großem Durchmesser | 26 | zylindrische Umfangsfläche |
| 6 | zweiter Arbeitsabschnitt von | | von 7 |
| | 18, zweite Manschette mit | 27 | vordere Öffnung von 5 |
| | kleinem Durchmesser | 28 | rückseitige Öffnung von 5 |
| 7 | Führungsstift | 29 | vordere Öffnung von 6 |
| 8 | Achse von 5, zweite Achse | 30 | rückseitige Öffnung von 6 |
| 9 | Schwingungsrichtung in 3 | 31 | vordere Öffnung |
| 10 | Schwingungsrichtung an 4 | 32 | rückseitiges Ende von 7 |
| 11 | Schwingungsrichtung an 5 | 33 | Unterkiefer |
| 12 | Schwingungsrichtung an etwa | 34 | Oberkiefer |
| | sechs | 35 | Mundöffnung |
| 13 | Hohlraum in 4 | 36 | Kreuzungsbereich von 18 |
| 14 | Hohlraum in 6 | 37 | vorbereitete Bohrung in 33 |
| 15 | Hohlraum in 5 | 38 | Boden von 37 |
| 16 | Achse von 4, erste Achse | 39 | poröser Wandbereich von 37 |
| 17 | Achse von 6, dritte Achse | 40 | Befestigungsöffnung für 18 |
| 18 | Sonotrode | | in 3 |
| 19 | distale Kante von 5 | 41 | Sonotrodenebene |
| 20 | distale Kante von 6 | 42 | rückseitiges Ende von 4, |
| 21 | Kreuzungspunkt der | | Anschlussseite von 4 |
| | Elemente 4-6 | 43 | freies Ende von 4 |
| 22 | Materialverdickungen im | | |
| | Bereich des | α | Öffnungswinkel zwischen 8 |
| | Kreuzungsbereichs an 4 | | und 17, respektive 5 und 6 |
| 23 | Materialverdickungen im | β | Öffnungswinkel zwischen 16 |
| | und 17, respektive 4 und 6 | Dᵢ | Innendurchmesser von 5 |
| γ | Öffnungswinkel zwischen 8 und 16, respektive 4 und 5, | l | Länge von Arbeitsabschnitt von 6 |
| | erster Winkel | d | Außendurchmesser von 6 |
| L | Länge von Arbeitsabschnitt | dᵢ | Innendurchmesser von 6 |
| | von 5 | k | Länge von 4 zwischen 3 und |
| D | Außendurchmesser von 5 | | 36 |

## Patentansprüche

1. Sonotrode (18) zum Anschluss an einen Ultraschall-Schwingungsgeber (2,3) mit einem sich entlang einer ersten Achse (16) erstreckenden Anschlussabschnitt (4), der mit einem ersten Ende (42) am Schwingungsgeber (2,3) befestigt oder mit diesem verbunden ist, wobei die Sonotrode dazu eingerichtet ist, dass der Anschlussabschnitt (4) durch den Ultraschall-Schwingungsgeber (2, 3) im wesentlichen ausschließlich in eine Schwingung entlang der ersten Achse (16) versetzt wird,
**dadurch gekennzeichnet, dass**
am freien, dem ersten Ende (42) gegenüberliegenden Ende (43) des Anschlussabschnittes (4) in einem gemeinsamen Kreuzungsbereich (36) sowohl eine im wesentlichen zylindrische, erste Manschette (5) angeordnet ist, welche sich entlang einer zweiten Achse (8) erstreckt, als auch eine im wesentlichen zylindrische, zweite Manschette (6), welche sich entlang einer dritten Achse (17) erstreckt, wobei die erste Achse (16) mit der zweiten Achse (8) einen ersten Winkel (γ) im Bereich von 100-140° einschließt, und wobei die erste Achse (16) mit der dritten Achse (17) einen zweiten Winkel (β) im Bereich von 100-140° einschließt, wobei die drei Achsen (8, 16,17) im wesentlichen in einer Sonotrodenebene (41) angeordnet sind.

2. Sonotrode (18) nach Anspruch 1, **dadurch gekennzeichnet, dass** das Verhältnis von Außendurchmesser (d, D) zur sich entlang der zweiten Achse (8) respektive der dritten Achse (17) erstreckenden Länge (1, L) der beiden Manschetten (5, 6) im Bereich von 1:2-1:10, vorzugsweise im Bereich von 1:3-1:6 liegt.

3. Sonotrode (18) nach einem der vorhergehenden Ansprüche, **dadurch gekennzeichnet, dass** der erste Winkel (γ) und/oder der zweite Winkel (β) im Bereich von 110-130°, vorzugsweise im Bereich von 115-125°, insbesondere vorzugsweise im Bereich von 118-122° liegen.

4. Sonotrode (18) nach einem der vorhergehenden Ansprüche, **dadurch gekennzeichnet, dass** der erste Winkel (γ) und der zweite Winkel (β) bis auf ein abweichen von nicht mehr als 5°, vorzugsweise von nicht mehr als 2° gleich sind, und dass bevorzugtermassen die zweite Achse (8) und die dritte Achse (17) einen dritten Winkel (α) einschließen, welcher im Bereich von 100-140°, insbesondere im Bereich von 110-130°, insbesondere bevorzugt im Bereich von 105-125° liegt.

5. Sonotrode (18) nach einem der vorhergehenden Ansprüche, **dadurch gekennzeichnet, dass** sowohl die erste Manschette (5) als auch die zweite Manschette (6) als Kreiszylinder mit einer kreiszylindrischen Außenfläche ausgebildet sind, und wobei der Außendurchmesser (D) der ersten Manschette (5) größer ist als der Außendurchmesser (d) der zweiten Manschette, wobei er vorzugsweise 1.1-3 mal, insbesondere vorzugsweise 1.2 - 2 mal größer ist, und wobei bevorzugtermassen das Verhältnis von Außendurchmesser (d, D) zur sich entlang der zweiten Achse (8) respektive der dritten Achse (17) erstreckenden Länge (1, L) der beiden Manschetten (5, 6) im wesentlichen gleich ist.

6. Sonotrode (18) nach einem der vorhergehenden Ansprüche, **dadurch gekennzeichnet, dass** die erste Manschette (5) und/oder die zweite Manschette (6), vorzugsweise beide, eine zentrale axiale zylindrische Ausnehmung (14,15) aufweisen, welche zum jeweiligen freien Ende unter Ausbildung einer vorderen Öffnung (27,29) offen liegen, wobei vorzugsweise die im Bereich dieser vorderen Öffnung (27,29) dadurch gebildete umlaufende Kante der jeweiligen Manschette (5, 6) konisch zulaufend ausgebildet ist, und wobei bevorzugtermassen die Ausnehmung (14,15) eine Durchgangsbohrung mit konstantem Innendurchmesser (Di, di) ist, welche an der jeweiligen Stelle des Kreuzungsbereichs (36) über eine jeweilige rückseitige Öffnung (28,30) zugänglich ist.

7. Sonotrode (18) nach einem der vorhergehenden Ansprüche, **dadurch gekennzeichnet, dass** sowohl Anschlussbereich (4) als auch beide Manschetten (5, 6) als röhrenförmige Hohlzylinder ausgebildet sind, welche im Kreuzungsbereich (36) zusammenlaufen respektive miteinander verbunden sind, indem durch beide Manschetten (5, 6) von jeweils beiden Seiten zugängliche Durchgangsöffnungen (27-30) vorhanden sind wobei vorzugsweise am freien Ende (43) des Anschlussbereichs (4) eine vordere Öffnung vorhanden ist, die weiterhin vorzugsweise durch ein Abschlusselement verschlossen sein kann.

8. Sonotrode (18) nach einem der vorhergehenden Ansprüche, **dadurch gekennzeichnet, dass** die erste Manschette (5) und die zweite Manschette (6) eine, insbesondere bezüglich einer durch die erste Achse (16) und eine Ebenennormale zur Sonotrodenebene (41) aufgespannte Spiegelebene symmetrische Massenverteilung aufweisen, wobei zur Kompensation von Unterschieden in der Länge (L, 1) und/oder im Durchmesser (D,d) und/oder im Innendurchmesser (Di, di) an der Sonotrode (18) im Kreuzungsbereich (36) Materialverdickungen (23,24) und/oder Materialausnehmungen ausgebildet sind.

9. Sonotrode (18) nach einem der vorhergehenden Ansprüche, **dadurch gekennzeichnet, dass** sie aus einem metallischen Werkstoff besteht, vorzugsweise ausgewählt aus der Gruppe bestehend aus: Aluminium, Eisen, Titan, Stahl sowie diese in der Hauptsache enthaltende oder aus diesen bestehende Legierungen, wobei vorzugsweise die Sonotrode einstückig und aus einem einzigen Werkblock bearbeitet ausgebildet ist, oder wobei vorzugsweise die Sonotrode aus ineinander geschraubten Elementen gebildet wird, indem der Anschlussabschnitt (4) und/oder der Kreuzungsbereich (36) und/oder die erste Manschette (5) und/oder die zweite Manschette (6) miteinander verschraubt werden.

10. Sonotrode (18) nach einem der vorhergehenden Ansprüche, **dadurch gekennzeichnet, dass** beide Manschetten (5, 6) eine Länge (L, 1) im Bereich von 5-50 mm, vorzugsweise im Bereich von 10-25 mm aufweisen und einen Durchmesser im Bereich von 2-15 mm, vorzugsweise im Bereich von 2.5-10 mm, insbesondere vorzugsweise im Bereich von 2.5-7 mm.

11. Sonotrode (18) nach einem der vorhergehenden Ansprüche, **dadurch gekennzeichnet, dass** am ersten Ende (42) des Anschlussabschnitts (4) eine Schnittstelle zur Befestigung am Ultraschall-Schwingungsgeber (2, 3) vorgesehen ist, vorzugsweise in Form eines formschlüssigen und/oder kraftschlüssigen Kupplungsbereiches, insbesondere bevorzugt in Form eines Gewindes, eines Flansches, einer Nut oder eines Bajonettverschlusses.

12. Sonotrodenwerkzeug (1) mit einem Ultraschall-Schwingungsgeber (2, 3) und einer Sonotrode (18) nach einem der vorhergehenden Ansprüche, wobei bevorzugtermassen der Schwingungsgeber einen als Handgriff ausgebildeten Konverter (2) umfasst sowie einen Booster (3), wobei die Sonotrode am Booster befestigt ist oder mit diesem einstückig ausgebildet ist.

13. Sonotrodenwerkzeug (1) nach Anspruch 12, **dadurch gekennzeichnet, dass** es als Handwerkzeug für den medizinischen Bereich, insbesondere für den Implantatbereich ausgelegt ist, insbesondere für den dentalen Bereich.

14. Verwendung einer Sonotrode (18) nach einem der vorhergehenden Ansprüche 1-11 oder eines Sonotrodenwerkzeugs nach Anspruch 12 zur Amelioration einer Ausnehmung, insbesondere einer Ausnehmung in einem porösen, löchrigen und durch die Ausnehmung freigelegte Hohlräume aufweisenden Material, wobei eine der beiden zylindrischen Manschette (5, 6) mit zylindrischer Mantelfläche mit einem Aussendurchmesser und mit einer zentralen Ausnehmung (14,15) zur Aufnahme eines Führungsstiftes (7), eingesetzt wird, wobei der Führungsstift (7) dazu vorgesehen ist, vor dem Anlegen von mechanischer Energie im wesentlichen bis auf den Boden der Ausnehmung eingeführt zu sein, wobei der Führungsstift (7) im Bereich seines dem Boden der Ausnehmung zugewandten Endes von einer Ameliorationshülse aus einem mit mechanischer Energie verflüssigbaren Material umschlossen wird, wobei die zylindrische Mantelfläche der Ameliorationshülse im wesentlichen den gleichen Aussendurchmesser aufweist wie die Manschette (5,6), und wobei der Führungsstift (7) in der zentralen Ausnehmung (14,15) derart verschieblich aufgenommen ist, dass die Manschette (5,6) bei Anlegen von mechanischer Energie relativ zum Führungsstift (7) in Richtung zum Boden der Ausnehmung unter Verflüssigung und seitlicher und/oder longitudinaler Verdrängung des Materials der Ameliorationshülse verschoben werden kann.

## Claims

1. Sonotrode (18) for connection to an ultrasonic vibration transducer (2,3) comprising a connecting portion (4), which extends along a first axis (16) and is attached by a first end (42) to the vibration transducer (2,3) or is connected thereto, wherein the the sonotrode is adapted such that the connecting portion (4) is made to vibrate almost exclusively along the first axis (16) by the ultrasonic vibration transducer (2,3),
**characterized in that**
at the free end (43) of the connecting portion (4) opposite from the first end (42), in a common crossing region (36) both a substantially cylindrical, first sleeve (5), which extends along a second axis (8), and a substantially cylindrical, second sleeve (6), which extends along a third axis (17), are arranged, wherein the first axis (16) forms a first angle (γ) in the range of 100-140° with the second axis (8), and wherein the first axis (16) forms a second angle (β) in the range of 100-140° with the third axis (17), wherein the three axes (8,16,17) are arranged substantially in one sonotrode plane (41).

2. Sonotrode (18) according to claim 1, **characterized in that** the ratio of the outside diameter (d,D) to the length (l,L) along the second axis (8) respectively the third axis (17), is in the range of 1:2-1:10, preferably in the range 1:3-1:6 for the two sleeves (5,6).

3. Sonotrode (18) according to one of the preceding claims, **characterized in that** the first angle (γ) and / or the second angle (ß) is in the range of 110-130°, preferably in the range 115-125°, particularly preferably in the range of 118-122°.

4. Sonotrode (18) according to any one of the preceding claims, **characterized in that** the first angle (γ) and the second angle (ß) are the same up to a deviation of not more than 5°, preferably not more than 2°, and **in that** preferably the second axis (8) and the third axis (17) form a third angle (α), which lies in the range of 100-140°, in particular in the range 110-130°, particularly preferably in the range of 105-125°.

5. Sonotrode (18) according to one of the preceding claims, **characterized in that** both the first sleeve (5) and the second sleeve (6) are designed as a circular cylinder with a circular cylindrical outer surface, and wherein the outside diameter (D) of the first sleeve (5) is greater than the outside diameter (d) of the second sleeve (6), being preferably 1.1-3 times, more preferably 1.2-2 times greater, and preferably wherein the ratio of the outside diameter (d,D) to the length (1,L) along the second axis (8) respectively to the third axis (17) is substantially the same for the two sleeves (5,6).

6. Sonotrode (18) according to one of the preceding claims, **characterized in that** the first sleeve (5) and / or the second sleeve (6), preferably both, has a central axial cylindrical recess (14,15), which is open to the respective free end to form a front opening (27,29), wherein preferably the encircling edge of each sleeve (5,6) formed in the area of this front opening (27,29) is designed preferably conically tapered, and wherein preferably the recess (14,15) is a through-bore with a constant inside diameter (Di,di) which is accessible at the respective position of the crossing region (36) through a respective rear opening (28,30).

7. Sonotrode (18) according to one of the preceding claims, **characterized in that** both the connecting portion (4) and the two sleeves (5,6) are designed as tubular hollow cylinders, which converge and are connected to each other in the crossing region (36), through-openings (27-30) accessible through both sleeves (5,6) from both sides respectively being provided, wherein preferably at the free end (43) of the connecting portion (4) a front opening is provided, which furthermore preferably can be closed by a closing element.

8. Sonotrode (18) according to one of the preceding claims, **characterized in that** the first sleeve (5) and the second sleeve (6) have a symmetrical mass distribution, in particular with respect to a mirror plane arranged through the first axis (16) and a plane normal to the sonotrode plane (41), wherein, for compensating for differences in the length (L,l) and / or in the diameter (D,d) and / or in the inside diameter (Di,di), material thickenings (23,24) and / or material recesses are formed at the sonotrode (18) in the crossing region (36).

9. Sonotrode (18) according to one of the preceding claims, **characterized in that** it is made of a metallic material, preferably selected from the group consisting of: aluminum, iron, titanium, steel, and principally containing these or composed of these alloys, wherein preferably the sonotrode is formed in one piece and machined from a single work block, or wherein preferably the sonotrode is formed by threaded elements screwed together, the connecting portion (4) and / or the crossing region (36) and / or the first sleeve (5) and / or the second sleeve (6) being screwed together.

10. Sonotrode (18) according to one of the preceding claims, **characterized in that** the two sleeves (5,6) have a length (L,l) in the range of 5-50 mm, preferably in the range of 10-25 mm and a diameter in the range of 2-15 mm, preferably in the range of 2.5-10 mm, in particular preferably in the range of 2.5-7 mm.

11. Sonotrode (18) according to one of the preceding claims, **characterized in that** at the first end (42) of the connecting portion (4) an interface for attachment to the ultrasonic vibration transducer (2,3) is provided, preferably in the form of a form-fit and / or force-fit coupling region, and in particular preferably in the form of a thread, a flange, a groove or a bayonet catch.

12. Sonotrode tool (1) with an ultrasonic vibration transducer (2,3) and a sonotrode (18) according to one of the preceding claims, wherein preferably the vibration transducer comprises a handle-shaped converter (2) and a booster (3), wherein the sonotrode is fixed to the booster or is designed integrally therewith.

13. Sonotrode tool (1) according to claim 12, **characterized in that** it is designed as a hand tool for the medical area, especially for the area of implants, especially for the dental area.

14. Use of a sonotrode (18) according to one of the preceding claims 1-11, or of a sonotrode tool according to claim 12 for the amelioration of a recess, in particular a recess in a porous, foraminous, and through the recess exposed with hollow spaces material, wherein one of the two cylindrical sleeves (5,6) with a cylindrical lateral surface having an outside diameter and a central recess (14,15) for accomodating a guide pin (7) is used, wherein the guide pin (7) is provided to be inserted substantially down to the base of the recess before applying mechanical energy, wherein the guide pin (7) in the region of the end thereof facing the base of the recess is surrounded by an amelioration collar made from a material that can be liquefied by way of mechanical energy, wherein the cylindrical lateral surface of the amelioration collar substantially has the same outside diameter as the sleeve (5,6), and wherein the guide pin (7) is accommodated in the central recess (14,15) in a displaceable manner such that the sleeve (5,6) upon applying mechanical energy can be displaced relative to the guide pin (7) in the direction toward the base of the recess while liquefying and laterally and / or longitudinally displacing the material of the amelioration collar.

## Revendications

1. Sonotrode (18) à raccorder à un générateur d'oscillations ultrasonores (2, 3) avec une partie de raccordement (4) s'étendant le long d'un premier axe (16), qui est, par une première extrémité (42) fixée au générateur d'oscillations (2, 3) ou reliée à celui-ci, dans laquelle la sonotrode est conçue de telle manière que la partie de raccordement (4) soit mise en oscillation par le générateur d'oscillations ultrasonores (2, 3), essentiellement exclusivement le long du premier axe (16),
**caractérisée en ce qu'**aussi bien une première manchette (5) essentiellement cylindrique, qui s'étend le long d'un deuxième axe (8), qu'une deuxième manchette (6) essentiellement cylindrique, qui s'étend le long d'un troisième axe (17), est disposée à l'extrémité libre (43), opposée à la première extrémité (42), de la partie de raccordement (4) dans une région de croisement commune (36), dans laquelle le premier axe (16) forme avec le deuxième axe (8) un premier angle (γ) dans la plage de 100-140°, et dans laquelle le premier axe (16) forme avec le troisième axe (17) un deuxième angle (β) dans la plage de 100-140°, dans laquelle les trois axes (8, 16, 17) sont disposés essentiellement dans un plan de sonotrode (41).

2. Sonotrode (18) selon la revendication 1, **caractérisée en ce que** le rapport entre le diamètre extérieur (d, D) et la longueur (l, L) s'étendant le long du deuxième axe (8) respectivement du troisième axe (17) des deux manchettes (5, 6) se situe dans la plage de 1:2 à 1:10, de préférence dans la plage de 1:3 à 1:6.

3. Sonotrode (18) selon l'une quelconque des revendications précédentes, **caractérisée en ce que** le premier angle (γ) et/ou le deuxième angle (β) se situent dans la plage de 110-130°, de préférence dans la plage de 115-125°, en particulier de préférence dans la plage de 118-122°.

4. Sonotrode (18) selon l'une quelconque des revendications précédentes, **caractérisée en ce que** le premier angle (γ) et le deuxième angle (β) sont égaux à un écart près ne dépassant pas 5°, de préférence ne dépassant pas 2°, et **en ce que** le deuxième axe (8) et le troisième axe (17) forment de préférence un troisième angle (α), qui se situe dans la plage de 100-140°, en particulier dans la plage de 110-130°, en particulier de préférence dans la plage de 105-125°.

5. Sonotrode (18) selon l'une quelconque des revendications précédentes, **caractérisée en ce qu'**aussi bien la première manchette (5) que la deuxième manchette (6) sont réalisées sous forme de cylindre circulaire avec une surface extérieure cylindrique circulaire, et dans laquelle le diamètre extérieur (D) de la première manchette (5) est plus grand que le diamètre extérieur (d) de la deuxième manchette, dans laquelle il est 1,1-3 fois, en particulier de préférence 1,2-2 fois plus grand, et dans laquelle le rapport du diamètre extérieur (d, D) à la longueur (l, L) s'étendant le long du deuxième axe (8) respectivement du troisième axe (17) des deux manchettes (5, 6) est essentiellement égal.

6. Sonotrode (18) selon l'une quelconque des revendications précédentes, **caractérisée en ce que** la première manchette (5) et/ou la deuxième manchette (6), de préférence les deux, présente un évidement central axial cylindrique (14, 15), qui sont ouverts vers l'extrémité libre respective en formant une ouverture avant (27, 29), dans laquelle le bord périphérique ainsi formé de la manchette respective (5, 6) dans la région de cette ouverture (27, 29) avant est de préférence profilé en forme de cône, et dans laquelle l'évidement (14, 15) est de préférence un alésage de passage de diamètre intérieur constant (Di, di), qui est accessible à l'endroit respectif de la région de croisement (36) au moyen d'une ouverture arrière respective (28, 30).

7. Sonotrode (18) selon l'une quelconque des revendications précédentes, **caractérisée en ce qu'**aussi bien la région de raccordement (4) que les deux manchettes (5, 6) sont réalisées en forme de cylindres creux tubulaires, qui convergent respectivement ou sont connectés l'un à l'autre dans la région de croisement (36), par le fait qu'il se trouve des ouvertures de passage (27-30) accessibles respectivement par les deux côtés à travers les deux manchettes (5, 6), dans laquelle il se trouve de préférence à l'extrémité libre (43) de la région de raccordement (4) une ouverture avant, qui peut en outre être fermée par un élément de fermeture.

8. Sonotrode (18) selon l'une quelconque des revendications précédentes, **caractérisée en ce que** la première manchette (5) et la deuxième machette (6) présentent une distribution de masse symétrique, en particulier par rapport à un plan de symétrie passant par le premier axe (16) et une normale au plan de sonotrode (41), dans laquelle, pour la compensation de différences dans la longueur (L, 1) et/ou dans le diamètre (D, d) et/ou dans le diamètre intérieur (Di, di), des surépaisseurs de matière (23, 24) ou des évidements de matière sont formés sur la sonotrode (18) dans la région de croisement (36).

9. Sonotrode (18) selon l'une quelconque des revendications précédentes, **caractérisée en ce qu'**elle se compose d'un matériau métallique, choisi de préférence dans le groupe composé de: aluminium, fer, titane, acier ainsi que des alliages les contenant à titre principal ou constitués de ceux-ci, dans laquelle la sonotrode est de préférence réalisée en une seule pièce et usinée à partir d'un seul bloc de travail, ou dans laquelle la sonotrode est de préférence formée par des éléments vissés les uns dans les autres, par le fait que la partie de raccordement (4) et/ou la région de croisement (36) et/ou la première manchette (5) et/ou la deuxième manchette (6) sont vissées l'une à l'autre.

10. Sonotrode (18) selon l'une quelconque des revendications précédentes, **caractérisée en ce que** les deux manchettes (5, 6) présentent une longueur (L, 1) dans la plage de 5-50 mm, de préférence dans la plage de 10-25 mm, et un diamètre dans la plage de 2-15 mm, de préférence dans la plage de 2,5-10 mm, en particulier de préférence dans la plage de 2,5-7 mm.

11. Sonotrode (18) selon l'une quelconque des revendications précédentes, **caractérisée en ce qu'**il est prévu à la première extrémité (42) de la partie de raccordement (4) une interface pour la fixation au générateur d'oscillations ultrasonores (2, 3), de préférence sous la forme d'une région de couplage par emboîtement et/ou par adhérence, en particulier de préférence sous la forme d'un filet, d'une bride, d'une rainure ou d'un assemblage à baïonnette.

12. Outil sonotrode (1) avec un générateur d'oscillations ultrasonores (2, 3) et une sonotrode (18) selon l'une quelconque des revendications précédentes, dans lequel le générateur d'oscillations ultrasonores comprend de préférence un convertisseur (2) réalisé en forme de poignée ainsi qu'un amplificateur (3), dans lequel la sonotrode est fixée à l'amplificateur ou est réalisée d'une seule pièce avec celui-ci.

13. Outil sonotrode (1) selon la revendication 12, **caractérisé en ce qu'**il est conçu comme outil à main pour le domaine médical, en particulier pour le domaine des implants, en particulier pour le domaine dentaire.

14. Utilisation d'une sonotrode (18) selon l'une quelconque des revendications 1 à 11 ou d'un outil sonotrode selon la revendication 12 pour l'amélioration d'un évidement, en particulier d'un évidement dans un matériau poreux, troué ou présentant des cavités révélées par l'évidement, dans laquelle une des deux manchettes cylindriques (5, 6) présentant une surface latérale cylindrique avec un diamètre extérieur et avec un évidement central (14, 15) est utilisée pour la réception d'une tige de guidage (7), dans laquelle la tige de guidage (7) est prévue pour être introduite essentiellement jusqu'au fond de l'évidement avant l'application d'énergie mécanique, dans laquelle la tige de guidage (7) est entourée dans la région de son extrémité tournée vers le fond de l'évidement par une douille d'amélioration en un matériau liquéfiable avec de l'énergie mécanique, dans laquelle la surface latérale cylindrique de la douille d'amélioration présente essentiellement le même diamètre extérieur que la manchette (5, 6) et dans laquelle la tige de guidage (7) est logée de façon coulissante dans l'évidement central (14, 15), de telle manière que la manchette (5, 6) puisse, lors de l'application d'énergie mécanique, être glissée par rapport à la tige de guidage (7) en direction du fond de l'évidement avec liquéfaction et refoulement latéral et/ou longitudinal du matériau de la douille d'amélioration.
